**Europäisches Patentamt**

**European Patent Office**

**Office européen des brevets**

(11) Veröffentlichungsnummer : **0 141 361 B1**

# (12) EUROPÄISCHE PATENTSCHRIFT

(45) Veröffentlichungstag der Patentschrift :
29.07.87

(21) Anmeldenummer : 84112715.2

(22) Anmeldetag : 22.10.84

(51) Int. Cl.⁴ : **C 07 C 87/60**, C 07 C103/44

(54) Verfahren zur Herstellung von 4-Nitro-1,3-diaminobenzol.

(30) Priorität : 04.11.83 DE 3339922

(43) Veröffentlichungstag der Anmeldung :
15.05.85 Patentblatt 85/20

(45) Bekanntmachung des Hinweises auf die Patenterteilung : 29.07.87 Patentblatt 87/31

(84) Benannte Vertragsstaaten :
CH DE FR GB LI

(56) Entgegenhaltungen :
DE-A- 2 724 362
US-A- 1 963 597
CHEMICAL ABSTRACTS, Band 46, Nr. 1, Januar 10, 1952, Spalten 106-107, A.M. KHALETSKII et al.: "Synthesis of piazthiole (2,1,3-benzothiadiazole) and its derivatives"

(73) Patentinhaber : BAYER AG
Konzernverwaltung RP Patentabteilung
D-5090 Leverkusen 1 Bayerwerk (DE)

(72) Erfinder : Schimpf, Rolf, Dr.
Winand-Rossi-Strasse 40
D-5090 Leverkusen 1 (DE)
Erfinder : Richter, Bernd, Dr.
Am Katterbach 13
D-5060 Bergisch-Gladbach 2 (DE)
Erfinder : Gleinig, Harald, Dr.
Eichholzer Weg 100
D-5068 Odenthal (DE)
Erfinder : Gomm, Walter, Dr.
Johann-Burum-Strasse 21
D-5060 Bergisch-Gladbach (DE)

EP 0 141 361 B1

**Beschreibung**

Die Erfindung betrifft ein Verfahren zur Herstellung von 4-Nitro-1.3-diaminobenzol durch Diacetylierung von 1.3-Diaminobenzol, Nitrierung des so erhaltenen N,N'-Diacetyl-1.3-diaminobenzols und Hydrolyse des bei der Nitrierung gebildeten 4-Nitro-N,N'-diacetyl-1.3-diaminobenzols.

Es ist bekannt, daß 1.3-Diaminobenzol durch Einwirkung von Essigsäure oder Essigsäurederivaten in N,N'-Diacetyl-1.3-diaminobenzol übergeführt werden kann. Die Acetylierung wird durch längeres Erhitzen in Eisessig durchgeführt (Ber. 7, 1257 (1874)). Weiterhin ist bekannt, die Acetylierung in Toluol mit Acetylchlorid in Gegenwart von Pyridin auszuführen (J. Amer. Chem. Soc. 59, 2003 (1937)). Auch die Umsetzung mit Acetylchlorid in Gegenwart von Na-acetat (Zh. Obs. Khim. 21, 1643 (1951)) bzw. mit Acetanhydrid in wäßrigem Medium ist beschrieben (J. Gen. Chem. (UdSSR) 14, 812 (1944)). Ferner ist die Reaktion mit überschüssigem Acetanhydrid (2 Mol pro Mol Amin) beschrieben (US 3. 639. 358).

Es ist darüber hinaus bekannt, trockenes N,N'-Diacetyl-1.3-diaminobenzol in Eisessig mit rauchender Salpetersäure (Ber. 7, 1259 (1874)) oder in gekühlter konzentrierter Salpetersäure (Ber. 30, 1912 (1897)) bzw. in sogenannten Mischsäuren (J. Chem. Soc. 87, 1941 (1905)) zu nitrieren.

Schließlich ist für die Hydrolyse des dabei entstandenen 4-Nitro-N,N'-diacetyl-1.3-diaminobenzols das Erhitzen in Sodalösung (J. Chem. Soc. 87, 1941 (1905)) bzw. in Natron- oder Kalilauge (Ber. 7, 1259 (1874)) vorgeschlagen worden.

Sämtliche vorstehend erwähnten Verfahrensschritte sind mit einer Reihe von Nachteilen behaftet.

So erfordert beispielsweise die Acylierung in organischen Lösungsmitteln aus sicherheitstechnischen Gründen einen relativ hohen apparativen Aufwand, in wäßrigen Medien hingegen einen hohen Verbrauch an Acylierungsmittel. Bei der erwähnten Acetylierung mit überschüssigem Acetanhydrid wird ebenfalls viel Acylierungsmittel (2 Mol pro Mol Amin) verwendet.

Ziemlich aufwendig ist auch die Zwischentrocknung des zumeist auf Wasser ausgetragenen Acylierungsproduktes.

Nachteilig bei der abschließenden alkalischen Hydrolyse ist die durch die Bildung von Nebenprodukten resultierende unbefriedigende Reinheit der Verfahrensprodukte.

Es wurde nun ein Verfahren zur Herstellung von 4-Nitro-1.3-diaminobenzol durch Diacetylierung von 1.3-Diaminobenzol, Nitrierung des N,N'-Diacetyl-1.3-diaminobenzols und Hydrolyse des dabei gebildeten 4-Nitro-N,N'-diacetyl-1.3-diaminobenzols gefunden, das dadurch gekennzeichnet ist, daß man zur Nitrierung ein wasserfeuchtes N,N'-Diacetyl-1.3-diaminobenzol einsetzt.

Bei der praktischen Durchführung der einzelnen Verfahrensschritte geht man im allgemeinen wie folgt vor :

Geschmolzenes 1.3-Diaminobenzol wird bei Temperaturen von etwa 120 bis 200 °C, bevorzugt bei 120 bis 180 °C, mit etwa 1,3 bis 1,8 Mol, vorzugsweise mit 1,6 bis 1,75 Mol, ganz besonders bevorzugt mit 1,7 Mol Acetanhydrid pro Mol Diamin umgesetzt, dem gegebenenfalls etwa 0,1 bis 1 Mol Essigsäure pro Mol Diamin zugesetzt wird.

Dabei wird das entstandene Reaktionswasser, gegebenenfalls in Verbindung mit Essigsäure, bei Normaldruck im angegebenen Temperaturbereich abdestilliert. Es ist auch möglich, die Destillation bei Unterdruck (etwa 40 bis 250 mbar) durchzuführen.

Nach vollständigem Umsatz des 1.3-Diaminobenzols wird das Reaktionsgemisch in ein auf ca. 60 bis 80 °C, bevorzugt 70 bis 75 °C, erwärmtes Essigsäure/Wasser-Gemisch eingetragen. Das Essigsäure/Wasser-Gemisch hat einen Gehalt von vorzugsweise 6 bis 10 Gew.-% Essigsäure. Dabei verwendet man bevorzugt die bei der Acetylierung abdestillierte Essigsäure.

Während des Eintragens des Gemisches in Essigsäure/Wasser geht das Reaktionsprodukt zunächst vollständig in Lösung und kristallisiert beim Abkühlen aus. Das auskristallisierte N,N'-Diacetyl-1.3-diaminobenzol wird anschließend direkt isoliert.

Das erhaltene feuchte Produkt mit einem Wassergehalt von etwa 8 bis 25 Gew.-%, bevorzugt 10 bis 15 Gew.-%, wird dann in die nachfolgende Nitrierung eingesetzt.

Die Nitrierung des so erhaltenen wasserfeuchten N,N'-Diacetyl-1,3-diaminobenzols wird üblicherweise so durchgeführt, daß das Produkt in konzentrierter Schwefelsäure mit einer Mischung aus Salpetersäure und Schwefelsäure im Temperaturbereich von etwa — 10 °C bis + 10 °C, bevorzugt 0 bis + 5 °C, umgesetzt wird.

Dabei wird das wasserfeuchte Diacetylierungsprodukt vorsichtig in die etwa 2,6 bis 4-fache, bevorzugt 2,7 bis 2,9-fache, Gewichtsmenge konzentrierter Schwefelsäure (ca. 96 bis 100 %ige) eingetragen und darin bei niederen Temperaturen (etwa — 10 bis + 10 °C, bevorzugt 0 bis + 5 °C) mit einer Mischung aus Salpetersäure und Schwefelsäure, die etwa 25 bis 60, bevorzugt 30 bis 35, Gew.-% an Salpetersäure enthält, nitriert.

Man verwendet zweckmäßigerweise etwa 0,99 bis 1,01 Mol, bevorzugt 1,0 Mol, Salpetersäure pro Mol Diacetylierungsprodukt. Grundsätzlich ist es auch möglich, ein anderes Nitrierverfahren zu wählen, z. B. in konzentrierter Schwefelsäure mit konzentrierter Salpetersäure (vgl. z. B. Houben-Weyl, Methoden der organischen Chemie, Band X, 1 (1971), S. 479 ff) zu nitrieren.

Nach beendeter Reaktion wird das Reaktionsgemisch auf Wasser unter Kühlung auf etwa 0 bis 20 °C, bevorzugt 10 bis 15 °C, ausgetragen und das ausgefallene Nitrierungsprodukt durch Filtration isoliert.

Für die anschließende hydrolytische Abspaltung der Acetylgruppen kann die Nitroverbindung vorteilhafterweise ebenfalls in Form der Feuchtpaste eingesetzt werden. Zu diesem Zweck wird diese Paste in die etwa 1 bis 10-fache, vorzugsweise 2 bis 3-fache Menge etwa 1 n bis 5 n, vorzugsweise 1,8 bis 2,2 n Salzsäure eingetragen und etwa eine Stunde bei etwa 80 bis 95 °C gerührt.

Aus der dabei erhaltenen salzsauren Lösung kann das 4-Nitro-1.3-diaminobenzol nach dem Abkühlen auf Raumtemperatur durch Neutralstellen und Filtration isoliert werden.

Da das 4-Nitro-1.3-diaminobenzol im allgemeinen als Diazokomponente zum Aufbau von Azofarbstoffen eingesetzt wird, ist eine derartige Zwischenisolierung der Aminbase nicht erforderlich. Es empfiehlt sich vielmehr, die bei der Hydrolyse gewonnene salzsaure Lösung unmittelbar gegebenenfalls nach Verringerung der Salzsäurekonzentration durch Zugabe von Eis mit Natriumnitrit zu diazotieren und die erhaltene Diazoniumsalzlösung mit geeigneten Kupplungskomponenten zu vereinigen.

### Beispiel 1 Herstellung von N,N'-Diacetyl-1.3-diaminobenzol

150 g 1.3-Diaminobenzol ( = 1,39 Mol) werden in einem Dreihalskolben vorgelegt, auf 100 °C erhitzt und dabei aufgeschmolzen. Unter Rühren läßt man dann langsam 184 ml Essigsäureanhydrid = 200 g = 1,96 Mol zulaufen. Dabei steigt die Temperatur auf 120 bis 130 °C an. Man erhitzt 6 Stunden lang am Rücklauf auf 120 bis 130 °C. Dann erhöht man die Innentemperatur auf 180 °C und destilliert dabei Reaktionswasser und Essigsäure ab. Nach insgesamt 6 Stunden läßt man weitere 38 ml Acetanhydrid = 41,3 g zulaufen und hält die Reaktion weitere 2 Stunden am Rücklauf. Dann heizt man wiederum auf 180 °C und destilliert Reaktionswasser und Essigsäure ab.

Als Destillat erhält man insgesamt 53,1 g einer 74,5 %igen Essigsäure, das entspricht 39,5 g reiner Essigsäure.

Nach vollständigem Umsatz von 1.3-Diaminobenzol wird das Reaktionsgemisch auf ein Gemisch von 560 ml Wasser von 70 °C und Essigsäure-Destillat (s. o. ) gegeben. Die Temperatur steigt dabei auf ca. 83 °C und man erhält eine klare Lösung. Beim Abkühlen kristallisiert daraus das N,N'-Diacetyl-1.3-diaminobenzol aus.

Man filtriert das Produkt bei 20 °C ab.

Ausbeute

284,6 g einer 85 %igen Feuchtware = 241,9 g 100 %ig = 1,26 Mol, das sind 90,4 % der Theorie.

Gesamtverbrauch an Acetanhydrid

241,3 g = 2,36 Mol, das sind 1,70 Mol pro Mol Diamin.

### Beispiel 2 Herstellung von 4-Nitro-N,N'-diacetyl-1.3-diaminobenzol

225,9 g feuchtes N,N'-Diacetyl-1.3-diaminobenzol (85 %ig) = 192 g 100 %ig = 1 Mol wird unter Kühlung auf 10 bis 15 °C in 342 ml = 633 g konzentrierter Schwefelsäure eingetragen. Nach Abkühlung des Reaktionsgemisches auf 0 bis 5 °C werden innerhalb von 4 bis 5 Stunden 189 g einer Mischsäure, bestehend aus 33 % Salpetersäure und 67 % Schwefelsäure, hinzugetropft (das entspricht 0,99 Mol Salpetersäure). Die Nitrierung erfolgt bei 0 bis 5 °C, vorwiegend 5 °C. Nach einer Stunde Nachrühren bei 0 bis 5 °C gibt man das Nitriergemisch unter Kühlung auf 1 970 ml Wasser bei 10 bis 15 °C. Dabei fällt das Reaktionsprodukt aus. Anschließend wird es auf einer Nutsche abfiltriert und mit Wasser gewaschen, bis das ablaufende Wasser einen pH-Wert von 3 hat.

Ausbeute

774 g einer feuchten Paste (30 %ig), das sind 232,2 g 100 %ig = 0,98 Mol = 98 % der Theorie.

### Beispiel 3 Herstellung von 4-Nitro-1.3-diaminobenzol

710 g feuchtes, 30 %iges 4-Nitro-N,N'-diacetyl-1.3-diaminobenzol (= 213,3 g 100 %ig = 0,9 Mol) werden in einer Mischung von 1 985 ml Wasser und 532 ml 30 %iges Salzsäure eingetragen. Man erhitzt das Reaktionsgemisch auf 90 °C und hält es dann noch eine Stunde lang auf dieser Temperatur. Dabei erhält man eine klare Lösung.

Nach entsprechender Verdünnung mit Eiswasser kann anschließend mit NaNO$_2$-Lösung diazotiert werden.

Zur Isolierung des 4-Nitro-1.3-diaminobenzols wird mit 411 ml 50 %iger Natronlauge auf pH 9 gestellt und dabei gleichzeitig auf 25 °C abgekühlt. Man rührt eine Stunde bei 25 °C nach und filtriert dann ab.

Ausbeute

174,7 g Feuchtprodukt (70 %ig) = 122,3 g 100 %ig = 0,80 Mol, das sind 88,8 % der Theorie.

**Patentansprüche**

1. Verfahren zur Herstellung von 4-Nitro-1.3-diaminobenzol durch Diacetylierung von 1.3-Diaminobenzol, Nitrierung des N,N'-Diacetyl-1.3-diaminobenzols und Hydrolyse des dabei gebildeten 4-Nitro-N,N'-diacetyl-1.3-diaminobenzols, dadurch gekennzeichnet, daß zur Nitrierung ein wasserfeuchtes N,N'-Diacetyl-1.3-diaminobenzol eingesetzt wird.

2. Verfahren nach Anspruch 1, dadurch gekennzeichnet, daß ein N,N'-Diacetyl-1.3-diaminobenzol eingesetzt wird, das einen Wassergehalt von 8 bis 25 Gew.-% hat.

3. Verfahren nach Anspruch 1 und 2, dadurch gekennzeichnet, daß ein N,N'-Diacetyl-1.3-diaminobenzol eingesetzt wird, das einen Wassergehalt von 10 bis 15 Gew.-% hat.

4. Verfahren nach Ansprüchen 1 bis 3, dadurch gekennzeichnet, daß ein wasserfeuchtes N,N'-Diacetyl-1.3-diaminobenzol eingesetzt wird, das durch Umsetzung von geschmolzenem 1.3-Diaminobenzol mit Acetanhydrid, dem gegebenenfalls Essigsäure zugesetzt wurde, unter ständigem Abdestillieren des Reaktionswassers bei Temperaturen von 120 bis 200 °C erhalten wurde.

**Claims**

1. Process for the preparation of 4-nitro-1,3-diaminobenzene by diacetylating 1,3-diaminobenzene, nitrating the N,N'-diacetyl-1,3-diaminobenzene and hydrolysing the 4-nitro-N,N'-diacetyl-1,3-diaminobenzene thereby formed, characterised in that a water-moist N,N'-diacetyl-1,3-diaminobenzene is used for the nitration.

2. Process according to Claim 1, characterised in that an N,N'-diacetyl-1,3-diaminobenzene is used which has a water content of 8 to 25 % by weight.

3. Process according to Claim 1 and 2, characterised in that an N,N'-diacetyl-1,3-diaminobenzene is used which has a water content of 10 to 15 % by weight.

4. Process according to Claims 1 to 3, characterised in that a water-moist N,N'-diacetyl-1,3-diaminobenzene is used which has been obtained by reacting molten 1,3-diaminobenzene with acetic anhydride, to which acetic acid has optionally been added, while continuously distilling off the water of reaction, at temperatures from 120 to 200 °C.

**Revendications**

1. Procédé pour la fabrication de 4-nitro-1,3-diaminobenzène par diacétylation du 1,3-diaminobenzène, nitration du N,N'-diacétyl-1,3-diaminobenzène et hydrolyse du 4-nitro-N,N'-diacétyl-1,3-diaminobenzène ainsi formé, caractérisé en ce que l'on utilise pour la nitration un N,N'-diacétyl-1,3-diaminobenzène imprégné d'eau.

2. Procédé selon la revendication 1, caractérisé en ce que l'on utilise un N,N'-diacétyl-1,3-diaminobenzène ayant une teneur en eau de 8 à 25 % en poids.

3. Procédé selon les revendications 1 et 2, caractérisé en ce que l'on utilise un N,N'-diacétyl-1,3-diaminobenzène ayant une teneur en eau de 10 à 15 % en poids.

4. Procédé selon les revendications 1 à 3, caractérisé en ce que l'on utilise un N,N'-diacétyl-1,3-diaminobenzène imprégné d'eau, qui a été obtenu par réaction du 1,3-diaminobenzène fondu avec l'anhydride acétique, éventuellement additionné d'acide acétique, avec distillation continue de l'eau de réaction à des températures de 120 à 200 °C.